# EUROPÄISCHE PATENTSCHRIFT

(11) **EP 0 859 553 B1**
(45) Veröffentlichungstag und Bekanntmachung des Hinweises auf die Patenterteilung: **03.05.2000**
(21) Anmeldenummer: 96938961.8
(22) Anmeldetag: 04.10.1996
(51) Int. Cl.: A23J 1/14, A23L 1/211

(54) **VERFAHREN ZUR VERARBEITUNG PROTEINHALTIGER PFLANZEN**
METHOD OF PROCESSING PROTEIN-CONTAINING PLANTS
PROCEDE DE TRAITEMENT DE PLANTES CONTENANT DES PROTEINES

(30) Priorität: 05.10.1995 DE 19537037
(43) Veröffentlichungstag der Anmeldung: 26.08.1998
(73) Patentinhaber: Finanzconsul AG, 8039 Zürich (CH); FRAUNHOFER-GESELLSCHAFT ZUR FÖRDERUNG DER ANGEWANDTEN FORSCHUNG E.V., 80636 München (DE)
(72) Erfinder: JAEGGLE, Wolfgang, D-88212 Ravensburg (DE); SCHMITZ, Volker, D-88212 Ravensburg (DE); ISDEBSKI, Kai, D-88348 Saulgau (DE); LUCK, Thomas, D-80992 München (DE); BORCHERDING, Axel, D-80937 München (DE); WÄSCHE, Andreas, D-80797 München (DE); OEH, Rainer, 81541 München (DE)
(74) Vertreter: Roth, Klaus
(86) Internationale Anmeldenummer: DE9601915
(87) Internationale Veröffentlichungsnummer: WO9712524

(56) Entgegenhaltungen:
- EP-A- 0 276 013
- EP-A- 0 441 672
- WO-A-83/00419
- DE-C- 537 265
- DATABASE WPI Section Ch, Week 9610 Derwent Publications Ltd., London, GB; Class D13, AN 96-095928 XP002025584 & RU 2 035 162 C (ROSINTRANS ASSOC) , 20.Mai 1995
- DATABASE WPI Section Ch, Week 8508 Derwent Publications Ltd., London, GB; Class D13, AN 85-044681 XP002025585 & DD 214 527 A (AKAD WISSENSCHAFT DDR) , 17.Oktober 1984
- JOURNAL OF FOOD SCIENCE, Bd. 46, 1981, Seiten 912-919, XP002025583 LAWHON, MANAK, RHEE, RHEE, LUSAS: "Combining aqueous extraction and membrane isolation techniques to recover protein and oil from soybeans"

## Beschreibung

Die Erfindung betrifft ein Verfahren zur Verarbeitung proteinhaltiger Pflanzen nach dem Oberbegriff des Anspruchs 1.

Verschiedene Pflanzen, z. B. die verschiedenen Lupinenarten, weisen einen hohen Anteil an hochwertigen pflanzlichen Proteinen auf. Derartige Proteine können eine wertvolle Bereicherung bei der Produktion von Lebensmitteln und Futtermitteln darstellen. Da die meisten Pflanzen und insbesondere die genannten Lupinenarten neben den gewünschten hochwertigen Proteinen auch sogenannte antinutritive Stoffe, z. B. Bitterstoffe (Alkaloide), bestimmte Zuckerformen (Oligosaccharide) etc. enthalten, die teilweise sogar giftig sein können, müssen die Pflanzen oder deren Samen vor der Verwertung der Proteine vorbehandelt werden.

Bei unterschiedlichen alten Kulturvölkern (Ägypter, Griechen, Inkas etc.) wurden bereits Lupinensamen mit Wasser behandelt, um derartige antinutritive Stoffe, insbesondere die Bitterstoffe von Lupinen, zu extrahieren.

Die Verfahren zur Entbitterung von Lupinensamen wurden in der Vergangenheit weiterentwickelt und mit modernen Methoden verbessert. So wird beispielsweise in der EP 084 547 ein Verfahren zur Entbitterung von Lupinensamen auf der Basis von Wasser als Extraktionsmittel beschrieben. Diese Druckschrift stellt jedoch vornehmlich auf die Gewinnung der Bitterstoffe aus den Lupinensamen ab, die ebenfalls nutzvoll als Pflanzenschutzmittel oder Pflanzenstärkungsmittel verwendbar sind. Der gemäß diesem Verfahren entstehende proteinhaltige Filterkuchen wird getrocknet und als hochwertiges Nahrungs- und Futtermittel empfohlen. Zur Weiterverarbeitung dieses Filterkuchens enthält diese Druckschrift keine Anleitung. Es wird lediglich ohne weitere Angaben eine Aufkonzentrierung als Möglichkeit genannt.

Weiterhin ist mit der EP 449 396 ein Verfahren zur Herstellung einer proteinhaltigen Lupinenmilch angegeben. Dieses Verfahren nutzt im wesentlichen das aus der Verarbeitung von Sojapflanzen, aber auch von Lupinen altbekannte Verfahren, indem vorgequollene Lupinensamen zermahlen, mit Wasser verrührt und anschließend abgepresst werden (vgl. hierzu Richard Yu, Lupin Newsletter, Februar 1989, S. 33 ff.). Die dabei entstehende Lupinenmilch soll als Ausgangsbasis für die Lebensmittelherstellung dienen.

Dieses Verfahren weist jedoch den Nachteil auf, daß sämtliche wasserlöslichen antinutritiven Stoffe in der Lupinenmilch enthalten sind. Sofern mit diesem Verfahren eine Bitterlupine verarbeitet wird, ist die daraus entstehende Lupinenmilch ungenießbar, da ein hoher Anteil an Bitterstoffen, d. h. Alkaloiden, in der Lupinenmilch enthalten ist.

Auch bei der Verarbeitung von Süßlupinen ist dieses Verfahren von Nachteil, da hier ebenfalls antinutritive Stoffe, wie z. B. Oligosaccharide, Bitterstoffe, etc., in der Lupinenmilch enthalten sind, die im menschlichen Verdauungstrakt unangenehme Nebeneffekte bewirken, wie z. B. Flatulenzen.

In der Druckschrift DE 53 72 65 wird eine wässrige Extraktion von Galaktanen beschrieben, bei der auch Alkaloide extrahiert werden. Hierbei verbleibt jedoch eine gewisse Restmenge an Alkaloiden, so daß eine Verwendung dieses Verfahrens für die Nahrungsmittelproduktion nicht möglich ist.

Die Druckschrift EP 0 276 013 sowie die Druckschrift WO 83 00 419 beschreiben eine Alkaloidextraktion mit Wasser, wobei auf die Verarbeitung der Proteine nicht näher eingegangen wird.

In der Druckschrift EP 0 441 672 wiederum wird die Verwendung verschiedener selektiver Lösungsmittel zur differenzierten Extraktion verschiedener Bestandteile wie Alkaloide und Proteine des Ausgangsprodukts beschrieben. Bei diesem Verfahren werden große Mengen unterschiedlicher Lösungsmittel benötigt, was einen erheblichen Aufwand in der industriellen Anwendung bedeutet.

Die Druckschrift DD 21 45 27 beschreibt eine Wasserdampfbehandlung des Rohmaterials aus pflanzlichen Samen vor der Extraktion der einzelnen Bestandteile, wobei bezüglich der nachfolgenden Extraktion der Einzelbestandteile und insbesondere auch der Auswirkungen der Wasserdampfbehandlung auf dieses anschließende Verfahren keine Aussagen getroffen werden.

Außerdem gibt der Stand der Technik keinerlei Hinweise auf die Nutzung weiterer wertvoller Inhaltsstoffe der zu verarbeitenden proteinhaltigen Pflanzen, die beispielsweise in Form von Ballaststoffen vorliegen können.

Die Erfindung hat daher die Aufgabe, ausgehend von dem vorgenannten Stand der Technik ein Verfahren zur Verarbeitung proteinhaltiger Pflanzen, insbesondere von Lupinen, vorzuschlagen, mittels dem Proteine und weitere insbesondere für die Ernährung wertvolle Stoffe so aufbereitet werden, daß diese ohne unerwünschte Nebenwirkungen für die Herstellung von Nahrungsmittel und Futtermittel, aber auch für andere Zwecke, z.B. als technische Rohstoffe, vewendbar sind.

Diese Aufgabe wird durch die kennzeichnenden Merkmale des Anspruchs 1 gelöst.

Durch die in den Unteransprüchen genannten Maßnahmen sind vorteilhafte Weiterbildungen und Ausführungen der Erfindung möglich.

Dementsprechend werden bei einem erfindungsgemäßen Verfahren nach einer Inaktivierung von Enzymen wenigstens zwei Extraktionen durchgeführt, wobei in einer Extraktion antinutritive Inhaltsstoffe, z. B. Alkaloide, Oligosacharide, etc., der Pflanzen bzw. der Pflanzenbestandteile abgetrennt werden und in der anderen Extraktion Proteine extrahiert werden.

Durch dieses Verfahren ist es möglich, eine mit Proteinen angereicherte Fraktion zu gewinnen, die problemlos im Lebensmittelbereich verwendbar ist, da antinutritive Stoffe extrahiert sind. Je nach Ausgangspflanze kann jedoch auch die Fraktion der antinutritiven Stoffe, z. B. als Pflanzenschutzmittel, Pflanzenstärkungsmittel, in der Medizin, o.ä., weiterverwendet werden. Auch die dritte aus den beiden Extraktionen hervorgehende Fraktion kann bei bestimmten Ausgangspflanzen, wie Lupinen oder dergleichen, wertvolle Inhaltstoffe, z. B. Ballaststoffe, Vitamine, etc., beinhalten.

Die Inaktivierung solcher Enzyme wird thermisch durch Wärmeeinwirkung bewirkt. Durch die Kombination der Einwirkung von Wärme und Feuchtigkeit wird die Inaktivierung von Enzymen verbessert. Daher wird die Inaktivierung bevorzugt durch Blanchieren mit Wasserdampf durchgeführt.

Dieses Dämpfen bewirkt als weiteren Vorteil, daß durch den Dampf die in Hohlräumen, wie Poren etc., des Rohproduktes vorhandene Luft verdrängt wird. Bringt man anschließend dieses Rohprodukt in eine Umgebung von kaltem Extraktionsmittel, so kondensiert der in diesen Hohlräumen befindliche Wasserdampf und das Extraktionsmittel wird in diese Hohlräume eingezogen. Das Rohmaterial wird so gewissermaßen mit Extraktionsmittel imprägniert. Es kommt somit zu einer Verbesserung der Oberflächenbenetzung mit dem Extraktionsmittel, wodurch die Effizienz der Extraktion gesteigert wird. Bevorzugt finden diese Vorgänge in einem geschlossenen System, d. h. einem geschlossenen Reaktionsbehälter statt.

Vorzugsweise werden die Pflanzen und/oder die Pflanzensamen vor den Extraktionen zerkleinert, um die Oberfläche, an der das oder die Extraktionsmittel angreifen können, zu vergrößern. Zur Erhöhung der Ausbeute empfiehlt es sich, die Pflanzen und/oder Pflanzensamen soweit zu zerkleinern, z. B. zu mahlen, bis eine grießförmige Struktur erreicht wird. Gute Ergebnisse wurden mit einem Grieß erzielt, der Korngrößen zwischen 200 und 600 Mikrometer aufweist.

Da das so vorliegende Rohprodukt aus den Pflanzen bzw. deren Samen u. a. auch Fette und entsprechende Enzyme zur Aufspaltung dieser Fette, sogenannte Lipoxygenasen enthält, empfiehlt es sich, dieses Rohprodukt vor der ersten Extraktion einer Behandlung zu unterziehen, bei der die genannten Enzyme inaktiviert werden. Hierdurch wird verhindert, daß im Rohprodukt bzw. in den aus dem beschriebenen Verfahren hervorgehenden Verfahrensprodukten bei entsprechender Lagerung eine Oxidation der vorhandenen ungesättigten Fettsäuren stattfindet. Eine solche Oxidation führt zu einem ranzigen Geschmack, was eine Verwendung im Lebensmittelbereich beeinträchtigen würde. Auch der Keimeintrag in die verwendete Anlage wird durch die vorgeschlagene Behandlung erheblich reduziert.

Um das Verhältnis von Proteinen zu Ballaststoffen bereits im Vorfeld vor der Durchführung der erfindungsgemäßen Extraktionen zu verbessern, empfiehlt es sich, lediglich die Pflanzensamen zu verarbeiten. Durch Schälen der Samen vor dem Zerkleinern kann bereits zu diesem Zeitpunkt ein erheblicher Anteil von Ballaststoffen abgetrennt werden. Die Samenschalen selbst können insbesondere in vermahlener Form als Ballaststoff weiterverarbeitet werden, z. B. für die Verwendung im Lebensmittel- oder Futtermittelbereich. Denkbar wäre jedoch auch ein Einsatz auf technischem Gebiet, z. B. als Dämmaterial auf dem Bausektor, usw.

Das erfindungsgemäße Verfahren eignet sich besonders gut für die Verarbeitung von Lupinen. Es hat sich gezeigt, daß bei der Verarbeitung von Lupinen bzw. von Lupinensamen nahezu geschmacksneutrale Produkte gewonnen werden können, die sich durch hohe Anteile hochwertiger Proteine und hochwertiger, d. h. fermentierbarer Ballaststoffe auszeichnen. Das Verfahren eignet sich sowohl für die Verarbeitung von Bitterlupinen als auch von Süßlupinen, da auch die letzteren, insbesondere in Form von den bereits genannten Oligosacchariden, antinutritive Stoffe enthalten, die wie oben angegeben zur Verwendung in Lebensmitteln ungeeignet sind. Vorteilhaft ist bei der Verarbeitung der Bitterlupine, daß die bitterstoffhaltige Fraktion, die aus einer der beiden Extraktionen resultiert, wie bereits erwähnt zusätzlich als Pflanzenschutzmittel, Pflanzenstärkungsmittel, zu medizinischen Zwecken, etc. weiterverwendbar ist.

Da insbesondere Wasser als Extraktionsmittel für die Verwendung im Lebensmittelbereich hervorragend geeignet ist, werden die Extraktionen mit Wasser als Extraktionsmittel durchgeführt. Die Ausbeute der entsprechenden Extraktion kann durch eine Veränderung des pH-Wertes optimiert werden.

Hierbei wird der Umstand genutzt, daß bei einer Extraktion im sauren Milieu der wesentliche Anteil der vorhandenen grundsätzlich wasserlöslichen Proteine nicht in Lösung geht und somit im wesentlichen nur die antinutritiven Stoffe extrahiert werden.

Vorteilhafterweise werden beide Extraktionen bei Atmosphärendruck durchgeführt, da somit der Aufwand beim Erstellen und Betrieb einer entsprechenden Umgebung in Grenzen gehalten wird. Weiterhin wird wenigstens eine der beiden Extraktionen mit Kaltwasser, d. h. in einem Temperaturbereich zwischen 15°C und 25°C betrieben. Hierdurch werden mikrobiologische Aktivitäten, insbesondere ein unerwünschtes Bakterienwachstum, während des Verfahrens weitgehend vermindert.

Daher werden zwei Extraktionen nacheinander mit Wasser als Extraktionsmittel durchgeführt, wobei die erste Extraktion im sauren Milieu und die zweite Extraktion im alkalischen Milieu stattfindet. Vorzugsweise wird die erste Extraktion bei einem pH-Wert von 3,5 bis 5,5 durchgeführt, wobei besonders gute Ergebnisse zwischen 4,5 und 5,0 erzielt wurden. Bei der zweiten Extraktion empfiehlt es sich, mit einem pH-Wert größer als 7,5, vorzugsweise größer als 8 zu arbeiten. Durch gezielte Variation des pH-Wertes in der ersten und/oder zweiten Extraktion ist eine selektive Auswahl von bestimmten Proteinen möglich.

Aus der zweiten Extraktion resultieren somit zwei Fraktionen, wobei die eine proteinangereichert und die andere proteinabgereichert ist. Durch die Vorschaltung der ersten Extraktion zur Entfernung der antinutritiven Stoffe sind beide Fraktionen der Verarbeitung zum Nahrungsmittel zugänglich und verursachen insbesondere keine unerwünschten Nebenwirkungen, z. B. Flatulenzen.

Die Proteinausbeute läßt sich steigern, indem aus dem von der ersten sauren Extraktion vorliegenden Extrakt die dort in Lösung befindlichen Restbestandteile von Proteinen ausgefällt werden, z. B. durch Hitzefällung, Zugabe von Alkohol, usw. Durch anschließende Trennung, beispielsweise mittels einer Zentrifuge, erhält man ein proteinhaltiges Sediment, das zur weiteren Verarbeitung in die erste saure Extraktionsstufe zurückgeführt wird, durch die diesem Sediment ebenso wie dem der Extraktion zugeführten Rohprodukt etwaige Rückstände von Bitterstoffen oder sonstigen antinutritiven Stoffen entzogen werden. Anschließend wird das gesamte sowohl aus dem pflanzlichen Rohstoff sowie dem genannten Sediment hervorgehende Raffinat gemeinsam weiterverarbeitet.

Ebenso wie die vorgenannte Proteinfällung kann der Extrakt aus der ersten sauren Extraktion auch einer Ultrafiltration (u. U. in Kombination mit einer Proteinfüllung) unterzogen werden und der hieraus resultierende Rückstand wieder der ersten sauren Extraktion zugeführt werden.

Vorteilhafterweise werden eine oder beide der genannten Extraktionen mehrstufig durchgeführt, wobei vorzugsweise ein Gegenstromverfahren eingesetzt wird. Derartige Extraktionsverfahren, bei dem stufenweise das Extraktionsmittel angereichert und im Gegenstrom das aus den jeweiligen Extraktionsstufen hervorgehende Raffinat abgereichert wird, sind bereits bekannt. In der ersten (sauren) Extraktion wurde mit einer neunstufigen Ausführung eine gute Ausbeute bei der Extraktion der antinutritiven Stoffe erzielt. Bei der zweiten (alkalischen) Extraktion, d. h. bei der Extraktion der Proteine hat sich ein zweistufiges Verfahren bewährt. Die Auftrennung der bei den Extraktionen vorliegenden Suspensionen wird bevorzugt mittels der Schwerkraft oder durch mechanische Trennung, z. B. durch Sedimentation, durch Zentrifugieren, durch Filtration, durch Pressen etc., durchgeführt.

Aus der vorbeschriebenen zweiten Extraktion gehen, wie oben angegeben, zwei Fraktionen hervor. Zum einen liegt eine proteinangereicherte Proteinmilch vor, während auf der anderen Seite ein Feststoffraffinat gewonnen wird, das bei einem gewissen Restanteil von Proteinen mit wertvollen, d. h. fermentierbaren Ballaststoffen angereichert ist. Dieses Raffinat kann unmittelbar im Lebensmittelbereich zur Ballaststoffanreicherung, beispielsweise in Backwaren, Getränken, Wurst, Frischprodukten oder dergleichen, verwendet werden.

Je nach Ausgangspflanze kann in der Proteinmilch ein bestimmter Fettanteil vorliegen. Es empfiehlt sich, diesen Fettanteil wenigstens teilweise abzutrennen, wobei eine mechanische Fettabtrennung, beispielsweise mittels einer Zentrifuge, im Lebensmittelbereich am unbedenklichsten erscheint, da hierbei keinerlei Chemikalien zugesetzt werden. Eine Fettabtrennung kann im übrigen bereits vor oder während einer oder mehreren Extraktionsstufen der ersten Extraktion und/oder auch vor oder während einer oder mehreren Extraktionsstufen der zweiten Extraktion durchgeführt werden.

Die andere Fraktion aus der zweiten Extraktion stellt eine proteinhaltige Milch dar. Aus dieser Proteinmilch können nun Frischprodukte auf pflanzlicher Basis, ähnlich wie Käse, Joghurt, Quark etc. durch weitere Verarbeitung gewonnen werden.

Die Proteinmilch kann jedoch auch zu einem Proteinkonzentrat bzw. -isolat weiterverarbeitet werden. Hierzu wird bevorzugt eine Proteinfällung durchgeführt, wofür beispielsweise eine Enzymfällung, eine Fällung durch Koagulationsmittel, eine Hitze- und/oder Säurefällung jeweils alleine oder in beliebiger Kombination miteinander in Frage kommt. Auch ein Membrantrennverfahren wäre für die Proteinisolation oder - konzentration anwendbar.

In einem vorteilhaften Ausführungsbeispiel der Erfindung werden die Proteine mit Säure ausgefällt, wobei ggf. im Anschluß daran eine Hitzefällung durchgeführt wird.

Als Koagulationsmittel kämen beispielsweise Ca SO₄, Mg SO₄, Na₂ SO₄ oder organische Stoffe wie Glucano- -Lacton, in Frage.

Nach der Trocknung der ausgefällten Bestandteile erhält man somit ein hochkonzentriertes proteinhaltiges Produkt. Dieses proteinhaltige Produkt wiederum eignet sich vorzüglich für den Einsatz im Lebensmittel- und Futtermittelbereich. Es kann jedoch auch in allen anderen Verwendungen von Proteinen, z. B. im Bereich der Pharmazie oder im Bereich der sogenannten technischen Proteine, insbesondere für die Herstellung von biologisch abbaubaren Kunststoffen oder Baustoffen, beispielsweise für Folien, Klebstoffe, Farben, etc., Verwendung finden.

Die Funktionalität, d. h. der Einfluß auf das chemische/physikalische Verhalten auf Systeme, z. B. Lebensmittelsysteme, des Proteinisolats bzw. -konzentrats sowie aller anderen beschriebenen Zwischen- oder Endprodukte kann bereits bei einer der beiden Extraktionen, vorzugsweise der zweiten, d.h. der alkalischen, Extraktion aus dem pflanzlichen Ausgangsstoff beeinflußt werden. Diese Beeinflussung der Funktionalität spielt bei der Anpassung der Proteine an die jeweiligen Anforderungen eine Rolle. Zur Änderung der Funktionalität können beispielsweise Zusatzstoffe beigesetzt werden oder Verfahrensparameter, wie Temperatur, pH-Wert, usw. variiert werden. Als Zusatzchemikalien kommen hierbei Oxidationsmittel, Substitutionsmittel, Metallionen (insbesondere zweiwertige, wie z. B. Ca⁺⁺) oder ähnliches in Betracht.

Weiterhin ist es möglich, sowohl die Proteinmilch als auch das daraus erhaltene Proteinkonzentrat bzw. -isolat durch Hydrolyse, z. B. durch enzymatische Hydrolyse, Säurehyrdolyse, etc. aufzuspalten. Das hieraus resultierende Hydrolysat, das ggf. noch einer Aufreinigung, Eindampfung und/oder Trocknung zu unterziehen ist, findet wiederum Verwendung in den unterschiedlichsten Bereichen, z. B. bei Lebensmitteln oder in der technischen Anwendung wie oben angeführt. Im Lebensmittelbereich können daraus unter anderem Sportlerpräparate oder Aufbaupräparate für Kranke hergestellt werden.

Durch die Wahl eines oder mehrerer bestimmter Enzyme und/oder Mikroorganismen können die Proteine selektiv gespalten werden. Falls eine Aufreinigung zur Gewinnung eines bestimmten Hydrolysats notwendig ist, so kann diese beispielsweise mittels eines Membrantrennverfahrens durchgeführt werden.

Zu erwähnen ist weiterhin, daß nach der oben angeführten Proteinfällung eine flüssige Molke vorliegt, die einen Restanteil von Proteinen beinhaltet, wobei diese Proteine höchst wasserlöslich sind. Auch diese höchst wasserlöslichen Proteine sind bei Bedarf z.B. durch ein Membrantrennverfahren, insbesondere durch Ultrafiltration zu gewinnen. Sie können entweder unmittelbar genutzt oder weiter verarbeitet werden. Man kann sie auch bei Bedarf dem durch die Proteinfällung gewonnenen Proteinisolat bzw. -konzentrat beigeben.

Ein Ausführungsbeispiel der Erfindung ist in der Zeichnung dargestellt und wird anhand der nachfolgenden Beschreibung näher erläutert.

Im einzelnen zeigen
- Fig. 1: ein Blockdiagramm, das die Vorbereitung der pflanzlichen Rohstoffe vor der Durchführung der erfindungsgemäßen Extraktionen veranschaulicht;
- Fig. 2: ein Blockdiagramm, aus dem ersichtlich ist, wie aus den pflanzlichen Rohstoffen in den zwei erfindungsgemäßen Extraktionen verschiedene Fraktionen gewonnen werden,
- Fig. 3: ein Blockdiagramm, aus dem die Verarbeitung eines aus einer Extraktion hervorgehenden Raffinats zum verkaufsfähigen Produkt hervorgeht,
- Fig. 4: ein Blockdiagramm das die Verarbeitung einer erfindungsgemäßen Proteinmilch darstellt und
- Fig. 5: ein Blockdiagramm, das die Herstellung von Frischprodukten veranschaulicht.

Der Verfahrensgang wird im folgenden an Hand der Verarbeitung von Lupinensamen beschrieben. Wie bereits angeführt, können auf die gleiche Weise auch andere proteinhaltige Pflanzen und insbesondere nicht nur Pflanzensamen verarbeitet werden.

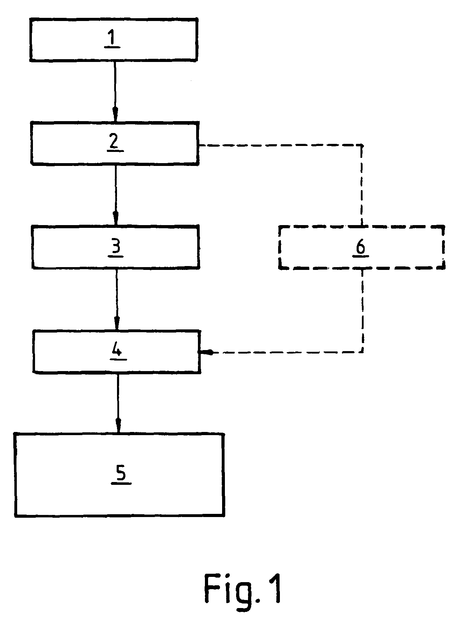

Das Verfahren gemäß Fig. 1 beginnt mit bereitgestellten Lupinensamen 1, die ggf. zuvor einer Reinigung unterzogen wurden, um Steine, Sand oder sonstigen Besatz abzutrennen. Dabei wird eine magnetische Abscheidung von Eisenbestandteilen, die unter Umständen durch maschinelle Aberntung, Transport und Lagerung in die Lupinensamen gelangen können, durchgeführt.

Die Lupinensamen 1 werden in einem Schälvorgang 2 geschält. Dies kann beispielsweise mittels eines Unter- oder Oberläuferschälgangs oder eines Prallschälers bewerkstelligt werden. Es hat sich als vorteilhaft erwiesen, die Lupinensamen vor der Schälung der Größe nach zu sortieren. Über einen Schalenseparator werden die Schalen von den Kernen getrennt und entweder zwischengelagert oder weiter verarbeitet. Die Kerne werden sodann in einem Mahlvorgang 3 mit zwischengeschalteter Sichtung solange gemahlen, z. B. mittels eines Walzenstuhls, bis die Korngröße im gewünschten Korngrößenspektrum liegt. Sollten im Lupinenmehl noch Schalenanteile enthalten sein, so können diese bei Bedarf über einen Windsichter separiert werden.

Die nun anschließende Blanchierung 4 dient zur Inaktivierung von Enzymen. Wie bereits erwähnt, wird hier insbesondere die Lipoxygenase inaktiviert, durch die ansonsten die im Lupinenmehl vorhandenen Fette wie o. a. oxidiert werden könnten. Die Blanchierung 4 wird mittels Dampf durchgeführt, wobei die Temperatur-Zeit-Belastung so gewählt ist, daß die Lupinenproteine ihre native Form beibehalten. Es hat sich bewährt, die Blanchierung bei ungefähr 92° C, einem Umgebungsdruck von 1 bar für eine Zeitdauer von ungefähr zwei Minuten durchzuführen. Die Blanchierung kann beispielsweise in einem kontinuierlich betriebenen Bandblancheur durchgeführt werden, bei dem ein kontinuierlicher und schonender Produktmassenstrom gewährleistet ist.

Nach der Blanchierung 4 steht das blanchierte Mehl für die erfindungsgemäßen Extraktionen 5 bereit.

Mit gestrichelten Linien ist in Fig. 1 weiterhin dargestellt, daß auch die abgetrennten Samenschalen einem Mahlvorgang 6 unterzogen werden können und ebenso wie die Kerne nach einer Blanchierung dem erfindungsgemäßen Extraktionsverfahren 5 zuführbar sind. Das gesamte, nach der Entschälung stattfindende Verfahren ist jedoch für die Kerne getrennt von den Schalen durchzuführen. Die Fig. 1 darf nicht dahingehend mißverstanden werden, daß lediglich die Mahlvorgänge 3 und 6 getrennt ablaufen würden. Aus der Verarbeitung der Schalen analog dem im folgenden für die Kerne beschriebenen Verfahren resultiert ein Ballaststoffkonzentrat oder -isolat, wie weiter unten noch erläutert wird.

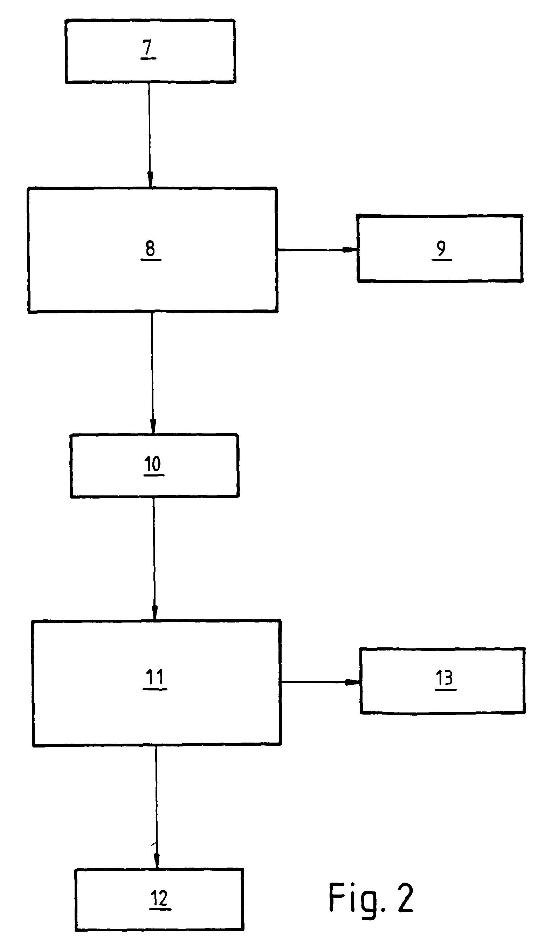

Die Verfahrensschritte gemäß Fig. 2 gehen aus von einem Rohprodukt 7, wie es aus der vorbeschriebenen Blanchierung 4 des Lupinenmehls aus den zermahlenen Kernen hervorgeht. Dieses Rohprodukt 7 wird einer ersten Extraktion 8 unterzogen. Diese erste Extraktion ist mit frischem Trinkwasser als Lösungsmittel im sauren Millieu durchführbar. Der pH-Wert soll sich hierbei in der Nähe des isoelektrischen Punkts der Lupinenproteine bewegen, d. h. in der Nähe von 4,5 liegen. Die Einstellung des pH-Wertes kann z.B. mittels Schwefelsäure (H₂SO₄) und/oder Phosphorsäure (H₃PO₄) vorgenommen werden.

Es hat sich gezeigt, daß durch ein solches Verfahren, insbesondere in Verbindung mit einer Gegenstromextraktion in mehreren Stufen, Alkaloide sowie weitere antinutritive Stoffe wie die bereits mehrfach erwähnten Oligosacharide bis auf unwesentliche Spuren aus dem Raffinat extrahiert werden können. Die Gegenstromextraktion kann in einer Rührkesselkaskade durchgeführt werden. Jede Stufe umfaßt hierbei einen Rührbehälter mit Rührwerk, einen Dekanter sowie eine Pumpe zur Speisung des Dekanters. Vorzugsweise werden die Rührbehälter mit einer Kühlvorrichtung verbunden, um dem Prozeß die durch die Dekanter eingetragene Energie zu entnehmen. Damit die Lupinenproteine ihre native Form beibehalten und die Löslichkeit der Proteine zur Erhöhung der Proteinausbeute niedriger gehalten wird, wird diese Extraktion mit Kaltwasser (z. B. bei einer Temperatur zwischen 15°C und 25°C) betrieben.

Die beispielsweise neunstufig aufgebaute Gegenstromextraktion geschieht so, daß das Lupinenmehl in der ersten Stufe und das Lösungsmittel (frisches Trinkwasser) in der neunten Stufe in die Extraktion eintritt. Das von antinutritiven Stoffen abgereicherte Mehl aus der ersten Stufe wird so dann in die zweite Stufe gegeben. Das geringfügig mit antinutritiven Stoffen angereicherte Lösungsmittel aus der neunten Stufe dient zur weiteren Abreichung des Mehls in der achten Stufe. Allgemein läßt sich sagen, daß jeweils das Mehl aus der Extraktionsstufe n der Extraktionsstufe n + 1 zugeführt wird, während das Lösungsmittel im Gegenstrom aus der Extraktionsstufe n der Extraktionsstufe n - 1 zugeführt wird. Der die antinutritiven Stoffe enthaltende Extrakt verläßt also die Extraktion aus der ersten Stufe, während das extrahierte Raffinat aus der neunten Stufe der Extraktion hervorgeht. In jeder dieser Stufen wird folglicherweise eine Mehl-Lösungsmittel-Suspension angerührt und wieder in ein Raffinat (feuchter Feststoff) und Extrakt (wäßriger Pflanzenextrakt) aufgetrennt.

In dem Extrakt 9 sind somit die wesentlichen Anteile der antinutritiven Stoffe enthalten, während mit dem Raffinat 10 ein pflanzliches Produkt vorliegt, das einen wertvollen Anteil sowohl an Proteinen als auch an verdaubaren Ballaststoffen aufweist. Dieses Raffinat 10 kann bereits zur Protein- und Ballaststoffanreicherung in Lebensmitteln oder Futtermitteln Verwendung finden. Die mögliche Weiterverarbeitung zu einem verkaufsfertigen Produkt wird weiter unten erläutert.

Das Raffinat 10 kann erfindungsgemäß mit einer zweiten Extraktion 11 weiter aufgeschlossen werden. Diese Extraktion wird im vorliegenden Ausführungsbeispiel ebenfalls mit Wasser als Lösungsmittel durchgeführt, wobei nunmehr auf ein alkalisches Milieu zu achten ist. Hierbei ist zu beachten, daß das Löslichkeitsmaximum für die Proteinextraktion in etwa im Bereich des pH-Werts 8,5 oder höher liegt.

Der pH-Wert kann jedoch, wie oben angegeben, variiert werden, um die Funktionalität der Proteine zur Anpassung an bestimmte Verwendungszwecke der Endprodukte des beschriebenen Verfahrens zu variieren. Eine Variation der Funktionalität der Endprodukte kann ebenfalls durch die Veränderung der Prozeßtemperatur oder durch Zugabe von Zusatzstoffen durchgeführt werden. Die genannten Maßnahmen zur Veränderung der Funktionalität können dabei von Stufe zu Stufe während der Extraktion verschieden sein.

Die Einstellung des pH-Wertes kann beispielsweise über Zugaben von NaOH, Ca(OH)₂ und/oder KOH geschehen, wobei die beiden letztgenannten Stoffe für die Verwendung in Lebensmitteln vorzuziehen sind, da Natrium ohnehin in hohem Maße in Nahrungsmitteln vorhanden ist.

Auch die zweite Extraktion ist mit Kaltwasser bei 15°C- 25°C durchführbar. Hierbei ist wiederum gewährleistet, daß die Proteine nicht denaturiert werden.

Im vorliegenden Ausführungsbeispiel wird die Proteinextraktion ebenfalls im Gegenstromverfahren jedoch mit zwei Stufen durchgeführt. Auch in der zweiten Extraktion können die oben angegebenen Maßnahmen zur Veränderung der Funktionalität entsprechend angewandt werden.

Nach Abschluß dieser zweiten Extraktion liegt ein Raffinat 12 vor, das einen hohen Anteil von Ballaststoffen enthält, während der Extrakt 13 eine mit Proteinen angereicherte Proteinmilch darstellt.

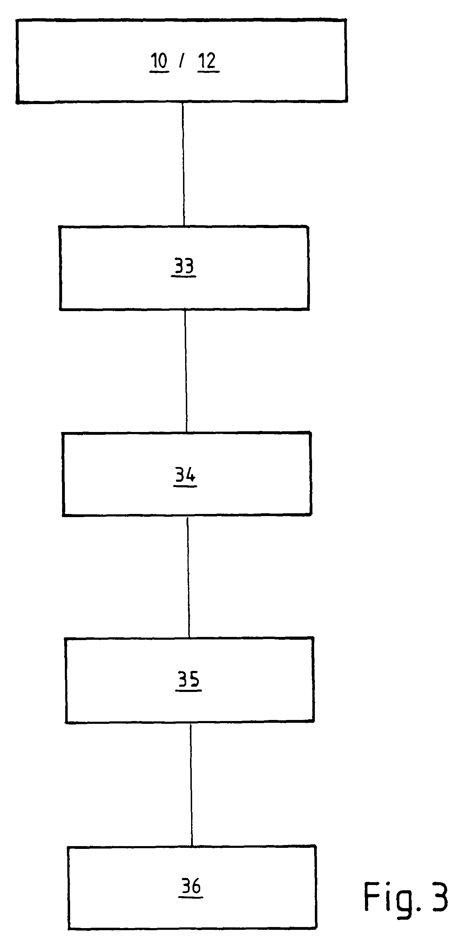

Sowohl das aus der ersten Extraktion 8 hervorgehende Raffinat 10 als auch das Raffinat 12 der zweiten Extraktion 11 kann bereits unmittelbar als Rohstoff für die Lebensmittel bzw. für die Futtermittelproduktion verwendet werden. Vorteilhafterweise werden die Raffinate den im folgenden an Hand von Fig.3 erläuterten Verfahrensschritte unterzogen, um zu einem verkaufsfertigen Produkt zu gelangen.

Ausgangspunkt ist jeweils das unmittelbar aus der entsprechenden Extraktion hervorgehende Raffinat 10 oder 12, dieses wird so dann getrocknet 33. Bei Bedarf können sich noch ein Mahlvorgang 34 und/oder eine Kühlungsphase 35 anschließen. Das so verarbeitete Endprodukt aus dem Raffinat 10 oder 12 kann anschließend beispielsweise in Pulver- oder Grießform verpackt 36, z. B. abgesackt werden. Somit liegen bereits zwei weiterverwertbare Endprodukte des erfindungsgemäßen Verfahrens vor, die sich durch ein unterschiedliches Verhältnis zwischen Ballaststoffgehalt und Proteingehalt unterscheiden.

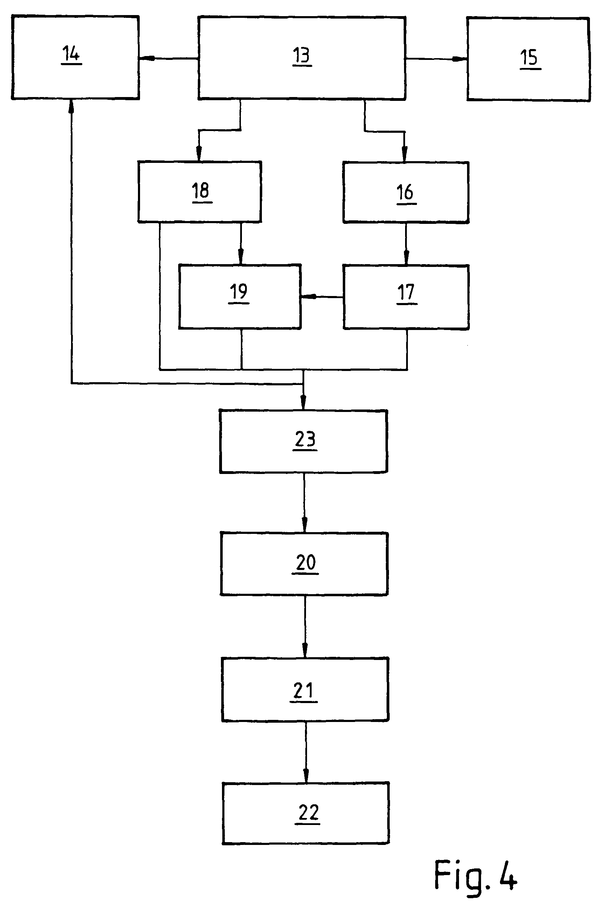

Die aus der zweiten (alkalischen) Extraktion 11 hervorgehende Proteinmilch 13 kann zur Herstellung verschiedenartigster proteinhaltiger Erzeugnisse dienen. In dem Prozeßablauf gemäß Fig. 4 sind in vereinfachter Darstellung die verschiedenen Verfahrenszweige veranschaulicht. Aus der Proteinmilch können beispielsweise diverse Fischprodukte 14, wie Joghurt, Quark, Käse und vieles andere mehr hergestellt werden. Auf den grundlegenden Verfahrensablauf hierbei wird weiter unten eingegangen.

Eine weitere Verarbeitungsmöglichkeit besteht darin, mittels enzymatischer Hydrolyse die Proteine der Proteinmilch aufzuspalten. Je nach Auswahl des Enzyms können hier funktionsspezifische Hydrolysate 15 gewonnen werden. Gegebenenfalls kann hierbei noch eine Aufreinigungsstufe zwischengeschaltet werden. Derartige durch Auswahl der jeweiligen Enzymen (Proteasen) selektiv gewonnen Peptide können unter anderem im Lebensmittelbereich, z. B. für Sportlerpräparate oder Krankenaufbaupräparate verwandt werden. Die Hydrolysate 15 können wiederum getrocknet als Pulver aber auch in flüssiger Form gehandelt werden.

Die Proteine der Proteinmilch 13 können auch in konzentrierter Form gewonnen werden. Hierzu müssen sie von der Molke getrennt werden. Zur Abtrennung der Proteine können diese beispielsweise einer Proteinfällung 16 unterzogen werden. Wie bereits erwähnt, kann die Proteinfällung 16 in einer Hitzefällung oder einer Säurefällung oder einer Kombination beider Fällungsarten bestehen. Nach der Abtrennung, beispielsweise mit Hilfe einer Zentrifuge liegt somit eine Molke, die nur noch höchst wasserlösliche Proteine enthält, sowie eine feuchte proteinhaltige Feststoffmasse vor. Eine andere Methode der Abtrennung der Proteine besteht in einem Membrantrennverfahren 18, z. B. einer Ultrafiltration. Auch hieraus ist unmittelbar ein feuchtes proteinhaltiges Produkt zu gewinnen.

Die aus beiden Verfahren, der Ultrafiltration 18 und insbesondere der Proteinfällung 16 mit Abtrennung 17 resultierende Molke kann wiederum einer Ultrafiltration 19 zur Gewinnung der höchstwasserlöslichen Proteine unterzogen werden. Diese werden gemäß dem vorliegenden Diagramm anschließend dem zuvor gewonnen feuchten Feststoffprodukt beigefügt. Sie können jedoch auch getrennt einer eigenen Verwendung zugeführt werden.

Aus der feuchten Feststoffmasse kann beispielsweise durch Auspressen wiederum ein Frischprodukt 14 in Form eines stichfesten Quarks gewonnen werden. In Fig. 4 ist dies durch eine zu den Frischprodukten 14 hinführende Abzweigung im Flußdiagramm veranschaulicht.

Das feuchte Proteinisolat bzw. -konzentrat das aus den vorangegangenen Verfahrensschritten hervorgeht, kann zu einem Trockenprodukt durch eine Trocknung 20, z.B. eine Sprühtrocknung mit nachgeschaltetem Fließbett, wobei bei Bedarf eine anschließende Kühlphase 21 vorgesehen wird, verarbeitet und als Verkaufsprodukt abgesackt werden 22. Gegebenenfalls werden an beliebiger Stelle, z.B. vor der Trocknung 20, zur Neutralisation des Proteinsisolats bzw. -konzentrats oder zur Veränderung der Funktionalität Zusatzstoffe 23 beigefügt.

Das aus dem Verfahrensablauf gemäß Fig. 4 resultierende Proteinisolat bzw. -konzentrat kann in vielfältiger Weise im Lebensmittel bzw. Futtermittelbereich verwendet werden. Natürlich können ohne weiteres auch aus dem Trockenprodukt 22 die oben angeführten Frischprodukte 14 hergestellt werden. Allerdings ist es in der Regel mit weniger Aufwand verbunden, die Frischprodukte 14 aus der Proteinmilch 13 unmittelbar herzustellen, da hierbei eine Trocknung entfällt. Ebenso steht das Trockenprodukt für eine Hydrolyse 15 zur Verfügung, wobei auch hier aus den gleichen Gründen die unmittelbare Verwendung der Proteinmilch 13 mit weniger Aufwand verbunden ist.

Außer dem Lebensmittel- und Futterbereich kann das Proteinisolat bzw. -konzentrat auch im Bereich der technischen Proteine verwendet werden. In Frage kommen hierfür, wie oben angegeben, Baustoffe oder Kunststoffe, z.B. Farben, Kleber, Folien, etc. Das Lupinenprotein verhält sich hierbei ähnlich wie das aus der Kuhmilch bekannte Kasein, das außer im Nahrungsmittelbereich ebenfalls bereits Verwendung für technische Produkte, beispielsweise bei Farben gefunden hat.

Weiterhin ist darauf hinzuweisen, daß auch die Lupinenmilch unter Umständen unmittelbar verwendbar ist. Ein Beispiel für die Notwendigkeit eines Milchersatzes ist die sogenannte Lactoseallergie, die hin und wieder auftritt, wobei die betroffenen Personen keinerlei lactosehaltigen Produkte aus Kuhmilch zu sich nehmen können. Hier kann unter Umständen mit der Proteinmilch Abhilfe geschaffen werden. Nicht nur die Proteinmilch als solches, sondern auch die bereits erwähnten Frischprodukte 14 auf Pflanzenbasis bieten in diesem Fall eine Alternative.

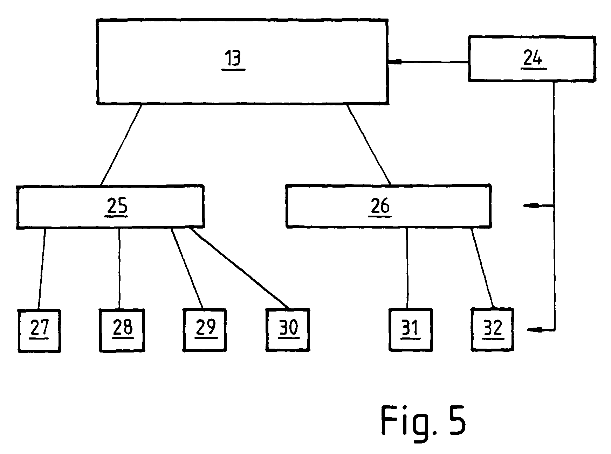

Die Frischprodukte 14 können (s. Fig. 5) durch Verfahren analog den aus der Kuhmilchverarbeitung bekannten Verfahren hergestellt werden. So kann die Proteinmilch 13 ggf. unter Zugabe von Zusatzstoffen 24, wie Mineralien oder Kohlehydrate entweder einer Milchsäurebakterienfällung 25 oder aber einer enzymatischen Fällung 26 (sogenannte Labfällung) unterzogen werden.

Aus der Milchsäurebakterienfällung 25 ergeben sich , ggf. wiederum unter Zugabe von weiteren Zusatzstoffen 24, z. B. wiederum Kohlehydrate und Mineralien, Produkte ähnlich wie Joghurt 27, Quark 28, Sauermilch 29 sowie verschiedene Weichkäse- oder Frischkäsesorten 30. Bei der enzymatischen Fällung entsteht ein Quark 31, der mit oder ohne Zusatzstoffe 24 zu Hartkäse 32 verarbeitbar ist.

Außer den genannten Lebensmittelformen sind auch alle aus der Verarbeitung von Sojabohnen bekannte Proteinprodukte herstellbar. Zu erwähnen sind hier beispielsweise Tofu- oder tofuähnliche Produkte oder auch sogenannter Tempeh, der durch einen Ansatz aus Proteinquark und Pilzsporen nach einer kontrollierten Brutzeit gewonnen werden kann.

Das Proteinisolat bzw. -konzentrat wurde bereits mit Erfolg als Austauschstoff bei der Fertigung von Schmelzkäse eingesetzt. Der üblicherweise bei der Schmelzkäse als Ausgangsstoff verwendete Gouda konnte zu erheblichen Teilen durch ein Proteinisolat bzw. -konzentrat gemäß der Erfindung ausgetauscht werden.

Die vorbeschriebenen Verfahrensstadien haben feste und flüssige Produkte hervorgebracht, die die unterschiedlichsten Protein-Ballaststoffverhältnisse aufweisen. Von den bislang beschriebenen Produkten war das Raffinat 12, das aus der zweiten erfindungsgemäßen Extraktion hervorgeht, das Ballaststoffreichste.

Unterzieht man jedoch die beim Schälvorgang 2 (Fig. 1) separierten Schalen den gleichen Verfahrensschritten wie vorbeschrieben und durch die gestrichelten Linien in Fig. 1 angedeutet, insbesondere wenigstens einer der beiden Extraktionen 8 und 11, so erhält man anstelle des Raffinats 12 ein Produkt, das fast ausschließlich Ballaststoffe enthält. Durch die erfindungsgemäßen Extraktionen, insbesondere die erste Extraktion 8, sind auch aus diesem Ballaststoffkonzentrat oder -isolat alle wesentlichen antinutritiven Stoffe weitgehend entfernt.

Bei der Verarbeitung der Schalen kann je nach Ausgangspflanze eine Extraktion in neutralem Milieu oder ohne gezielte pH-Wert Einstellung das gewünschte Ballaststoffkonzentrat bzw. -isolat ergeben, sofern kein wesentlicher Proteinanteil mehr in den Schalen enthalten ist.

Verständlicherweise kann bei der Vielfalt aller möglichen Endprodukte aus dem vorgestellten Verfahren zur Verarbeitung proteinhaltiger Pflanzen bzw. Pflanzensamen nicht jede spezielle Anwendung und jedes spezielle Endprodukt im Detail beschrieben werden. Wesentlich ist jedoch die Entfernung der antinutritiven Stoffe, wodurch sowohl die beschriebenen Proteinisolate bzw. -konzentrate als auch die Ballaststoffkonzentrate und -isolate bzw. Mischformen aus Ballaststoffen und Proteinen gut verdauliche Produkte darstellen.

## Patentansprüche

1. Verfahren zur Verarbeitung proteinhaltiger und alkaloidhaltiger Pflanzen oder deren Bestandteile, wie Lupinen oder dergleichen, und insbesondere von deren Pflanzensamen, dadurch gekennzeichnet, daß eine Inaktivierung von Enzymen durch Dämpfen vorgenommen wird und anschließend wenigstens zwei separate Extraktionen (5) mit Wasser als Extraktionsmittel durchgeführt werden, wobei in einer Extraktion (8) antinutritive Stoffe (9) und in der anderen Extraktion (11) Proteine extrahiert werden.

2. Verfahren nach Anspruch 1, dadurch gekennzeichnet, daß die Pflanzen und/oder Pflanzenbestandteile (1), wie Pflanzensamen, etc., zerkleinert (3) werden.

3. Verfahren nach einem der vorgenannten Ansprüche, dadurch gekennzeichnet, daß die Zerkleinerung (3) soweit durchgeführt wird, bis eine grießförmige Struktur vorliegt.

4. Verfahren nach einem der vorgenannten Ansprüche, dadurch gekennzeichnet, daß Pflanzensamen (1) geschält (2) werden.

5. Verfahren nach einem der vorgenannten Ansprüche, dadurch gekennzeichnet, daß Lupinen und/oder deren Bestandteile (1) verarbeitet werden.

6. Verfahren nach einem der vorgenannten Ansprüche, dadurch gekennzeichnet, daß in der ersten Extraktion (8) antinutritive Stoffe in einem sauren Milieu und in der zweiten Extraktion (11) Proteine in einem alkalischen Milieu extrahiert werden.

7. Verfahren nach einem der vorgenannten Ansprüche, dadurch gekennzeichnet, daß der pH-Wert bei der ersten (sauren) Extraktion (8) im Bereich von 3,5 bis 5,5 liegt.

8. Verfahren nach einem der vorgenannten Ansprüche, dadurch gekennzeichnet, daß der pH-Wert bei der zweiten (alkalischen) Extraktion (11) größer als 8 ist.

9. Verfahren nach einem der vorgenannten Ansprüche, dadurch gekennzeichnet, daß wenigstens eine Extraktion (8, 11) mehrstufig im Gegenstromverfahren durchgeführt wird.

10. Verfahren nach einem der vorgenannten Ansprüche, dadurch gekennzeichnet, daß eine mechanische Fettabtrennung durchgeführt wird.

11. Verfahren nach einem der vorgenannten Ansprüche, dadurch gekennzeichnet, daß aus der anfallenden Proteinmilch (13) Proteine ausgefällt und/oder durch ein Membranverfahren gewonnen werden.

12. Verfahren nach einem der vorgenannten Ansprüche, dadurch gekennzeichnet, daß die Funktionalität wenigstens eines der Endprodukte durch Zugabe von Zusatzstoffen, Auswahl der Prozeßtemperatur und/oder Einstellung des pH-Wertes während wenigstens einer Extraktionsstufe wenigstens einer der beiden Extraktionen (8, 11) variiert wird.

13. Ballaststoffangereichertes Pflanzenprodukt aus proteinhaltigen und alkaloidhaltigen Pflanzen oder deren Bestandteile, wie Lupinen oder dergleichen, und insbesondere von deren Pflanzensamen, dadurch gekennzeichnet, daß antinutritive Stoffe und Proteine wenigstens teilweise durch zwei Extraktionen gemäß Anspruch 1 extrahiert sind.

14. Proteinmilch auf Basis von proteinhaltigen und alkaloidhaltigen Pflanzen oder deren Bestandteile, wie Lupinen oder dergleichen, und insbesondere von deren Pflanzensamen, dadurch gekennzeichnet, daß die Proteinmilch nach zwei Extraktionen gemäß Anspruch 1 aus dem Extrakt der zweiten Extraktion gewonnen wird.

15. Proteinhaltiges Produkt, dadurch gekennzeichnet, daß es aus einer Proteinmilch gemäß Anspruch 14 herstellbar ist.

16. Proteinhydrolysat dadurch gekennzeichnet, daß es aus einer Proteinmilch nach Anspruch 14 und/oder aus einem proteinhaltigen Produkt nach Anspruch 15 herstellbar ist.

17. Frischprodukt nach einem der vorgenannten Ansprüche, dadurch gekennzeichnet, daß es wenigstens teilweise aus einer Proteinmilch gemäß Anspruch 14 und/oder einem proteinhaltigen Produkt gemäß Anspruch 15 herstellbar ist.

## Claims

1. Method ofprocessing protein-containing and alkaloid-containing plants of components thereof, such as lupins or the like, and in particular their seeds, characterised in that an inactivating of the enzymes is performed by means of vapours and afterwards at least two separate extractions (5) are performed with water as the extracting agent, whereby in one extraction (8) antinutritive substances (9) are extracted and in the other extraction (11) proteins are extracted.

2. Method according to claim 1, characterised in that the plants and/or plant components (1), such as seeds, etc. are reduced in size (3).

3. Method according to one of the preceding claims, characterised in that the reduction in size (3) is performed until a gritty structure is obtained.

4. Method according to one of the preceding claims, characterised in that seeds (1) are peeled (2).

5. Method according to one of the preceding claims, characterised in that lupins and/or their components (1) are processed.

6. Method according to one of the preceding claims, characterised in that in the first extraction (8) antinutritive substances are extracted in an acid environment and in the second extraction (11) proteins are extracted in an alkali environment.

7. Method according to one of the preceding claims, characterised in that the pH value in the first (acid) extraction (8) is in the range of 3.5 to 5.5.

8. Method according to one of the preceding claims, characterised in that the pH value of the second (alkali) extraction (11) is greater than 8.

9. Method according to one of the preceding claims, characterised in that at least one extraction (8, 11) is performed in several stages in a countercurrent procedure.

10. Method according to one of the preceding claims, characterised in that a mechanical fat separation is performed.

11. Method according to one of the preceding claims, characterised in that from the accumulated protein milk (13) proteins are precipitated and/or extracted in a membrane procedure.

12. Method according to one of the preceding claims, characterised in that the functionality of at least one of the end products is varied by the addition of additives, the choice of processing temperature and/or adjustment of the pH value during at least one extraction stage of at least one of the two extractions (8, 11).

13. Bulk-enriched plant product from protein-containing and alkaloid-containing plants or their components, such as lupins or the like and in particular their seeds, characterised in that antinutritive substances and proteins are at least partly extracted by means of two extractions according to claim 1.

14. Protein milk on the basis of protein-containing and alkaloid-containing plants or components thereof, such as lupins or the like, and in particular their seeds, characterised in that the protein milk is obtained after two extractions according to claim 1 from the extract of the second extraction.

15. Protein-containing product, characterised in that is can be made from a protein milk according to claim 14.

16. Protein hydrolyzate characterised in that it can be produced from a protein milk according to claim 14 and/or from a protein-containing product according to claim 15.

17. Fresh product according to one of the preceding claims, characterised in that it can be produced at least partly from a protein milk according to claim 14 and/or a protein-containing product according to claim 15.

## Revendications

1. Procédé pour traiter des plantes contenant des protéines et contenant des alcaloïdes ou leurs parties constitutives, comme des lupins ou analogues, et en particulier leurs graines,
caractérisé en ce qu'une inactivation des enzymes est réalisée par traitement à la vapeur et, ensuite, au moins deux extractions séparées (5) sont effectuées par de l'eau comme moyen d'extraction, les matières antinutritives (9) étant extraites dans une première extraction (8) et les protéines dans l'autre extraction (11).

2. Procédé selon la revendication 1,
caractérisé en ce que les plantes et/ou les parties constitutives des plantes (1), comme des graines, etc..., sont broyées (3).

3. Procédé selon une des revendications précitées,
caractérisé en ce que le broyage (3) est effectué jusqu'à atteindre une structure granuleuse.

4. Procédé selon une des revendications précitées,
caractérisé en ce que les graines (1) des plantes sont mondées (2).

5. Procédé selon une des revendications précitées,
caractérisé en ce que des lupins et/ou leurs parties constitutives (1) sont traités.

6. Procédé selon une des revendications précitées,
caractérisé en ce que, dans la première extraction (8), les matières antinutritives sont extraites dans un milieu acide et, dans la seconde extraction (11), les protéines sont extraites dans un milieu alcalin.

7. Procédé selon une des revendications précitées,
caractérisé en ce que la valeur de pH lors de la première (acide) extraction (8) se trouve dans la plage de 3,5 à 5,5.

8. Procédé selon une des revendications précitées,
caractérisé en ce que la valeur de pH lors de la seconde (alcaline) extraction (11) est supérieure à 8.

9. Procédé selon une des revendications précitées,
caractérisé en ce qu'au moins une extraction (8,11) est effectuée en plusieurs étapes dans le procédé à contre-courant.

10. Procédé selon une des revendications précitées,
caractérisé en ce qu'il est effectué une séparation mécanique des graisses.

11. Procédé selon une des revendications précitées,
caractérisé en ce que les protéines précipitent du lait ou dispersion de protéines (13) en résultant et/ou sont produites par un procédé à membrane.

12. Procédé selon une des revendications précitées,
caractérisé en ce que la fonctionnalité d'au moins un des produits finals est modifiée par ajout d'additifs, choix de la température du processus et/ou réglage de la valeur du pH pendant au moins une étape d'extraction d'au moins une des deux extractions (8,11).

13. Produit enrichi en matières de charge à partir de plantes contenant des protéines et contenant des alcaloïdes ou de leurs parties constitutives, comme des lupins ou analogues, et en particulier de leurs graines,
caractérisé en ce que des protéines et des matières antinutritives sont extraites au moins partiellement par deux extractions selon la revendication 1.

14. Lait ou dispersion de protéines sur la base de plantes contenant des protéines et contenant des alcaloïdes ou de leurs parties constitutives, comme des lupins ou analogues, en particulier de leurs graines,
caractérisé en ce que le lait ou la dispersion de protéines est produit après deux extractions selon la revendication 1 à partir de l'extrait de la seconde extraction.

15. Produit contenant des protéines,
caractérisé en ce qu'il peut être fabriqué à partir d'un lait ou d'une dispersion de protéines selon la revendication 14.

16. Hydrolysat de protéines,
caractérisé en ce qu'il peut être fabriqué à partir d'un lait ou d'une dispersion de protéines selon la revendication 14 et/ou à partir d'un produit comprenant des protéines selon la revendication 15.

17. Produit frais selon une des revendications précitées,
caractérisé en ce qu'il peut être fabriqué au moins partiellement à partir d'un lait ou d'une dispersion de protéines selon la revendication 14 et/ou d'un produit contenant des protéines selon la revendication 15.
